(19) Europäisches Patentamt
European Patent Office
Office européen des brevets

(11) Veröffentlichungsnummer: **0 325 927 A1**

# (12) EUROPÄISCHE PATENTANMELDUNG

(21) Anmeldenummer: **89100251.1**

(22) Anmeldetag: **13.01.86**

(51) Int. Cl.[4]: **C07D 319/06 , C07D 321/06 , C07D 319/08 , C07D 317/34 , C07D 317/72**

(30) Priorität: **23.01.85 DE 3502106**

(43) Veröffentlichungstag der Anmeldung: **02.08.89 Patentblatt 89/31**

(60) Veröffentlichungsnummer der früheren Anmeldung nach Art. 76 EPÜ: **0 190 568**

(84) Benannte Vertragsstaaten: **CH DE FR GB IT LI**

(71) Anmelder: **BAYER AG**

**D-5090 Leverkusen 1 Bayerwerk(DE)**

(72) Erfinder: **Mues, Peter, Dr.**
**Weihers Hecke 30**
**D-4100 Duisburg 46(DE)**
Erfinder: **Buysch, Hans-Josef, Dr.**
**Brandenburger Strasse 28**
**D-4150 Krefeld(DE)**

## (54) Verfahren zur Herstellung von cyclischen, aliphatischen Orthokolensäureestern sowie neue cyclische Orthokohlensäureester.

(57) Cyclische, aliphatische Orthokohlensäureester der Formel

EP 0 325 927 A1

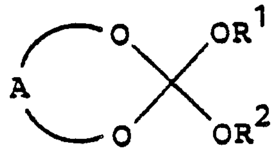

worin

A für 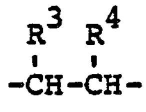, 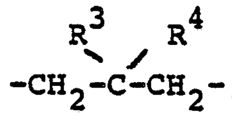, 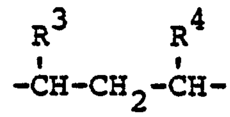, oder

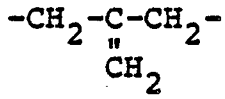 steht, wenn

$R^1$ und $R^2$ gemeinsam für 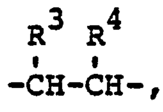, 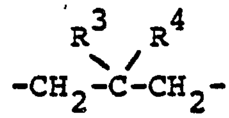,

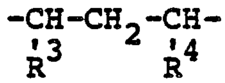 oder 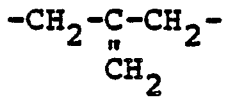 stehen,

oder

A für 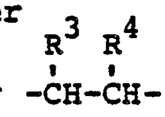, 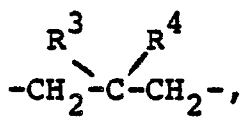, 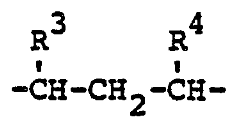,

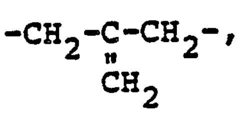, $-CH_2-CH=CH-CH_2-$ oder $-(CH_2)_4-$

steht, wenn
$R^1$ und $R^2$ einen Phenylrest, der unsubstituiert oder gegebenenfalls durch Chlor, Brom, eine Nitro-, Trifluormethyl-, Alkoxygruppe mit 1 bis 4 C-Atomen oder eine Carbalkoxygruppe mit 1 bis 4 C-Atomen ein- oder mehrfach substituiert ist, darstellen,
$R^3$ und $R^4$ gleich oder verschieden sein können und Wasserstoff, $C_1$-$C_4$-Alkyl oder Phenyl bedeuten oder gemeinsam für $-(CH_2)_m$ mit m = 2, 3, 4, 5 stehen,
werden hergestellt durch Umsetzung von Dichlormethanderivaten der Formel
$(R^5O)_2CCl_2$
worin
$R^5$ einen Phenylrest, der unsubstituiert oder gegebenenfalls durch Chlor, Brom, eine Nitro-, Trifluormethyl-, Alkoxygruppe mit 1 bis 4 C-Atomen oder eine Carbalkoxygruppe mit 1 bis 4 C-Atomen ein- oder mehrfach substituiert ist, darstellt,
mit aliphatischen Diolen der Formel
HO-A-OH
worin
A die obengenannte Bedeutung hat,
in Gegenwart stöchiometrischer Mengen organischer N-haltiger Basen.
Die cyclischen, aliphatischen Orthokohlensäureester stellen teilweise neue Verbindungen dar.

## Verfahren zur Herstellung von cyclischen, aliphatischen Orthokolensäureestern sowie neue cyclische Orthokohlensäureester

Die vorliegende Erfindung betrifft ein Verfahren zur Herstellung von cyclischen, aliphatischen Orthokohlensäureestern sowie neue cyclische Orthokohlensäureester.

Obwohl cyclische, aliphatische Orthokohlensäureester, insbesondere aliphatische Siroorthocarbonate, eine interessante Stoffklasse darstellen, sind bisher nur fünf Herstelungsmethoden für die Siroorthocarbonate bekannt geworden: Umsetzung von Di-thallium(I)-glykolaten mit $CS_2$ unter Abspaltung von Thalliumsulfid (J. Org. Chem. 37, 4198 (1972)); Reaktion von 0,0′-Bis-[tributylstannyl]-Derivaten von Glykolen mit $CS_2$ unter Abspaltung von Bis(tributylzinn)sulfid (J. Org. Chem. 35, 2347 (1970)); Umsetzung von Alkandiyldioxydibutylstannanen mit $CS_2$ (J. Org. Chem. 36, 1176 (1971)); Umsetzung von Orthokohlensäureestern mit Diolen unter Abspaltung von Alkohol (DE-OS 3 225 818; Synthesis 1984, 837) und Umsetzung von Expoxiden mit cyclischen fünf-oder sechsgliedrigen Carbonaten in Anwesenheit von Bortrifluoridetherat (DE-OS 3 406 049).

Die zuerst genannten drei Verfahren verlaufen zwar mit recht guten Ausbeuten, jedoch muß mit teuren Metallorganika und toxischem $CS_2$ gearbeitet werden. Bei den letztgenannten Verfahren wird das teure Tetraalkoxymethan als Ausgangsmaterial verwendet und die Abtrennung des Produkts vom neutralisierten Katalysator ist nicht unproblematisch und anscheinend Ausbeute bestimmend; die katalysierte Addition von Expoxiden an das Carbonat verläuft nur in sehr schlechten Ausbeuten.

Gegenstand der vorliegenden Erfindung ist ein Verfahren zur Herstellung von cyclischen Orthokohlensäureestern der Formel

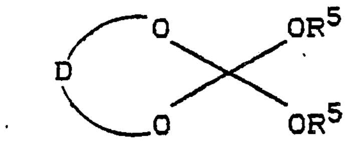 (VI),

worin

D für 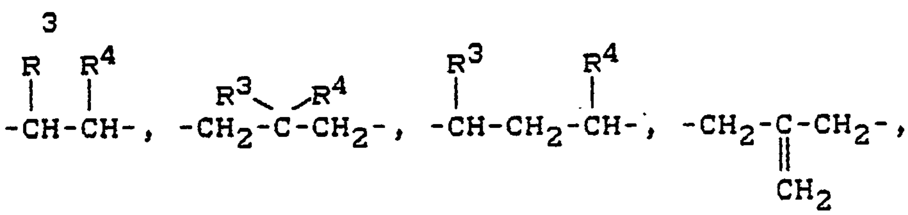

$-CH_2-CH=CH-CH_2-$ oder $-(CH_2)_4-$ steht,

$R^5$ einen Phenylrest, der unsubstituiert oder gegebenenfalls durch Chlor, Brom, eine Nitro-, Trifluormethyl-Alkoxygruppe mit 1 bis 4 C-Atomen oder eine Carbalkoxygruppe mit 1 bis 4 C-Atomen ein- oder mehrfach substituiert ist, darstellt,

und

$R^3$ und $R^4$ gleich oder verschieden sein können und Wasserstoff, $C_1$-$C_4$-Alkyl oder Phenyl bedeuten oder gemeinsam für $-(CH_2)_m-$ mit m = 2, 3, 4, 5 stehen,

das dadurch gekennzeichnet ist, daß man Dichlormethanderivate der allgemeinen Formel

$(R^5O)_2CCl_2$ (II),

worin

$R^5$ einen Phenylrest, der unsubstituiert oder gegebenenfalls durch Chlor, Brom, eine Nitro-, Trifluormethyl-, Alkoxygruppe mit 1 bis 4 C-Atomen oder eine Carbalkoxygruppe mit 1 bis 4 C-Atomen ein- oder mehrfach substituiert ist, darstellt,

mit aliphatischen Diolen der Formel

HO-D-OH (VII),

worin

D die obengenannte Bedeutung hat,

in Gegenwart stöchiometrischer Mengen an aliphatischen tertiären Aminen umsetzt oder für D = $-CH_2-$

$CH=CH-CH_2$-oder $-(CH_2)_4$- auch in Gegenwart stöchiometrischer Mengen an N-haltigen Basen, die den Stickstoff als Teil eines aromatischen Systems enthalten, umsetzt.

Gegenstand der vorliegenden Erfindung sind auch die cyclischen Orthokohlensäureester der Formel (VI), worin D, $R^3$, $R^4$ und $R^5$ die für Formel (VI) bereits genannte Bedeutung haben.

Als Verbindungen der allgemeinen Formel (II) seien beispielsweise genannt: Im Aromaten substituierte Diaryloxymethandichloride, wie Bis-(2,5-dichlorphenoxy)-methandichlorid, Bis-(4-chlorphenoxy)-methandichlorid, Bis-(4-nitrophenoxy)-methandichlorid, Bis-(3-nitrophenoxy)-methandichlorid, Bis-(3-methylphenoxy)-methandichlorid und Bis-(4-methylphenoxy)-methandichlorid und der unsubstituierte Grundkörper, das Diphenoxymethandichlorid. Besonders bevorzugt wird das Diphenoxymethandichlorid eingesetzt (II, $R^5$ = $C_6H_5$).

Als aliphatische Diole der Formel (VII), die zur Herstellung von cyclischen, aliphatischen Orthokohlen säureestern der allgemeinen Formel (VI) eingesetzt werden können, seien beispielsweise genannt: Glycol, Propandiol-1,2, Butandiol-2,3, Propandiol-1,3, 2-Phenylpropandiol-1,3, 1,1-Cyclohexandimethanol, 2,2-Dimethylpropandiol-1,3-, Butandiol-1,3, Pentandiol-2,4, 2-Butendiol-1,4 und Butandiol-1,4; besonders bevorzugt sind Glykol, Propandiol-1,2, Propandiol-1,3, 2-Methylenpropandiol-1,3, 2,2-Dimethylpropandiol-1,3, 2-Butendiol-1,4 und Butandiol-1,4. Die Diole werden in stöchiometrischen Mengen oder im Überschuß (etwa 10 bis 100 %iger Überschuß) eingesetzt; bevorzugt ist die Verwendung in stöchiometrischen Mengen.

Die erfindungsgemäße Umsetzung von Dichlormethanderivaten der allgemeinen Formel (II) mit den Diolen der allgemeinen Formel (VII) wird bevorzugt in inerten Lösungsmitteln durchgeführt. Geeignete Lösungsmittel hierfür sind beispielsweise Aromaten, wir Toluol und/oder Xylol, halogenierte Kohlenwasserstoffe, wie Methylenchlorid, Ethylenchlorid und/oder Ether, wie Diethylether, Tetrahydrofuran und/oder Anisol.

Das erfindungsgemäße Verfahren zur Herstellung der cyclischen, aliphatischen Orthokohlensäureester der Formel (VI) wird im allgemeinen bei Temperaturen von etwa 0 bis 60°C, bevorzugt 10 bis 40°C, durchgeführt.

Die Aufarbeitung des Reaktionsgemisches nach dem erfindungsgemäßen Verfahren zur Herstellung von cyclischen aliphatischen Orthokohlensäureestern kann beispielsweise so erfolgen, daß das entstandene Hydrochlorid (sofern es im gewählten Lösungsmittel unlöslich ist) abfiltriert oder wäßrig extraktiv entfernt wird.

Der nach Entfernung des Hydrochlorids und Lösungsmittels verbleibende Rückstand enthält das gewünschte Produkt; dieses kann entweder destillativ oder durch Kristallisation aus einem geeigneten inerten Lösungsmittel isoliert werden. Geeignete inerte Lösungsmittel sind beispielsweise Ester wie Ethylacetat oder Alkohole wie Methanol und/oder Ethanol.

Die bei der Umsetzung von Diolen der Formel (VII) mit dem Dichlormethanderivat (II) entstehende Salzsäure wird mit einem tertiären aliphatischen Amin gebunden. Beispielsweise können hierzu eingesetzt werden: Triethylamin 1,4-Diazabicyclo-(2,2,2)-octan, 1,5-Diazabicyclo-(4,3,0)-non-5-en, 1,5-Diazabicyclo-(5,4,0)-undec-7-en und N,N′-Dimethylpiperazin; besonders bevorzugt ist Triethylamin. Die tertiären Amine werden üblicherweise in stöchiometrischen Mengen eingesetzt.

Im Falle der Herstellung von neuen cyclischen Orthokohlensäureestern der Formel (VI) mit D = $-CH_2-CH=CHCH_2$-und - $(CH_2)_4$ kann die entstehende Salzsäure auch mit N-haltigen Basen gebunden werden, die den Stickstoff als Teil eines aromatischen Systems enthalten. Beispielsweise können hierzu verwendet werden: Pyridin, das gegebenenfalls auch ein- oder mehrfach durch Chlor, Brom, eine Nitro-, Trifluormethyl-, Alkoxygruppe mit 1 bis 4 Kohlenstoffatomen oder eine Carbalkoxygruppe mit 1 bis 4 Kohlenstoffatomen substituiert sein kann, Chinolin, Isochinolin, Chinazolin, Pyrimidin und N-Alkylimidazol, wie N-Methylimidazol. Die N-haltigen Basen werden in stöchiometrischen Mengen eingesetzt.

Aus den cyclischen Orthokohlensäureestern der Formel (VI) können im Sinne von US 4 070 347 und US 4 079 038 Kunststoffe und Überzüge, die auch zur Freisetzung von Wirkstoffen geeignet sind, hergestellt werden.

Beispiele 1 bis 4

0,12 Mol Diol der allgemeinen Formel (VII) udn 0,2 Mol Triethylamin wurden in 50 ml Dichlormethan vorgelegt, eine Lösung von 0,1 Mol Dichlordiphenoxymethan (II, $R^5$ = $C_6H_5$) in 50 ml Dichlormethan wurde unter Rühren bei 18 bis 20°C zugetropft. 2 Stunden Nachreaktion bei Raumtemperatur. Die Lösung wurde zweimal mit je 50 ml Wasser extrahiert, die organische Phase wurde über $Na_2SO_4$ getrocknet. Der nach Abziehen des Lösungsmittels im Wasserstrahlvakuum verbleibende Rückstand wurde aus Ethylacetat oder Methanol umkristallisiert.

1. Produkt: VI, D = $-(CH_2)_3-$, $R^1$, $R^2$ = $C_6H_5$; Fp. 96-97° C, Ausbeute: 95 %

2. Produkt: VI, D = $-CH_2-C(CH_3)_2-$, $R^1$, $R^2$ = $C_6H_5$; Fp, 99-100° C, Ausbeute: 95 %

3. Als Base wurde Pyridin für Triethylamin verwendet.

Produkt: VI, D = $-CH_2-CH=CH-CH_2-$, $R^1$, $R^2$ = $C_6H_5$; Fp. 71-72° C, Ausbeute: 95 %.

4. Als Base wurde Pyridin für Triethylamin verwendet.

Produkt: VI, D = $-(CH_2)_4-$, $R^1$, $R^2$ = $C_6H_5$; Fp. 82-84° C, Ausbeute: 95 %.

## Ansprüche

1. Verfahren zur Herstellung von cyclischen, aliphatischen Orthokohlensäureestern der Formel

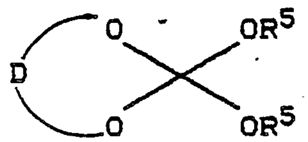

(VI),

worin

D für 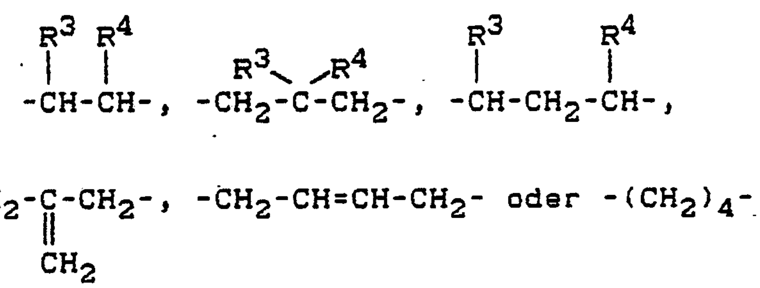

steht,

$R_5$ einen Phenylrest, der unsubstituiert oder gegebenenfalls durch Chlor, Brom, eine Nitro-, Trifluormethyl-, Alkoxygruppe mit 1 bis 4 C-Atomen oder eine Carbalkoxygruppe mit 1 bis 4 C-Atomen ein- oder mehrfach substituiert ist, darstellt,

$R^3$ und $R^4$ gleich oder verschieden sein können und Wasserstoff, $C_1$-$C_4$-Alkyl oder Phenyl bedeuten oder gemeinsam für $-(CH_2)_{\overline{m}}$ mit m = 2, 3, 4, 5 stehen,

dadurch gekennzeichnet, daß man Dichlormethanderivate der Formel

$(R^5O)_2CCl_2$ (II),

worin

$R^5$ einen Phenylrest, der unsubstituiert oder gegebenenfalls durch Chlor, Brom, eine Nitro-, Trifluormethyl-, Alkoxygruppe mit 1 bis 4 C-Atomen oder eine Carbalkoxygruppe mit 1 bis 4 C-Atomen ein- oder mehrfach substituiert ist, darstellt,

mit aliphatischen Diolen der Formel

HO-D-OH (VII),

worin

D die obengenannte Bedeutung hat,

in Gegegenwart stöchiometrischer Mengen an aliphatischen tertiären Aminen umsetzt, oder für D = $-CH_2CH=CH-CH_2-$ oder $-(CH_2)_4-$ auch in Gegenwart stöchiometrischer Mengen an N-haltigen Basen, die den Stickstoff als Teil eines aromatischen Systems enthalten, umsetzt.

2. Verfahren nach Anspruch 1, dadurch gekennzeichnet, daß man als tertiäre Amine Triethylamin, 1,4-Diazabicyclo-(2,2,2)-octan, 1,5-Diazabicyclo-(4,3,0)-non-5-en, 1,5-Diazabicyclo-(5,4,0)-undec-7-en oder N,N′-Di-methylpiperazin einsetzt.

3. Verfahren nach Anspruch 1, wobei D = $-CH_2-CH=CH-CH_2-$ oder $(CH_2)_4-$ ist, dadurch gekennzeichnet, daß man als N-haltige Basen, die den Stickstoff als Teil eines aromatischen Systems enthalten, Pyriolin, Chinolin, Isochinolin, Chinazolin, Pyrimidin oder N-Methylimidadol einsetzt.

4. Verfahren nach Ansprüchen 1 bis 3, dadurch gekennzeichnet, daß man die Umsetzung bei Temperaturen von 0 bis 60 °C duchführt.

5. Verfahren nach Ansprüchen 1 bis 4, dadurch gekennzeichnet, daß man die Umsetzung in Gegenwart von inerten, organischen Lösungsmitteln durchführt.

6. Verfahren nach Ansprüchen 1 bis 5, dadurch gekennzeichnet, daß man als inerte organische Lösungsmittel Toluol, Xylol, Methylenchlorid, Ethylenchlorid, Chlorbenzol, Diethylether, Tetrahydrofuran und/oder Anisol einsetzt.

7. Cyclische Orthokohlensäureester der Formel

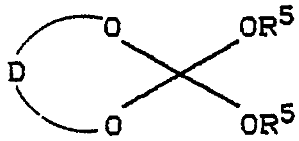 (VI),

worin

D für 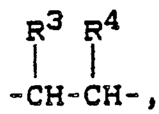, 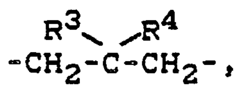, 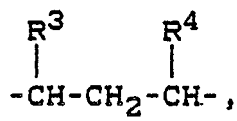,

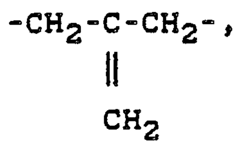, $-CH_2-CH=CH-CH_2-$ oder $-(CH_2)_4-$ steht,

$R^5$ einen Phenylrest, der unsubstituiert oder gegebenenfalls durch Chlor, Brom, eine Nitro-, Trifluormethyl-, Alkoxygruppe mit 1 bis 4 C-Atomen oder eine Carbalkoxygruppe mit 1 bis 4 C-Atomen ein- oder mehrfach substituiert ist, darstellt,
und
$R^3$ und $R^4$ gleich oder verschieden sein können und Wasserstoff, $C_1$-$C_4$-Alkyl oder Phenyl bedeuten oder gemeinsam für $-(CH_2)_m-$ mit m = 2, 3, 4, 5 stehen.

Europäisches Patentamt

# EUROPÄISCHER RECHERCHENBERICHT

Nummer der Anmeldung

EP 89 10 0251

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE-B-2 407 863 (L. GIVAUDAN)<br>* Ansprüche *<br>--- | 1,7 | C 07 D 319/06<br>C 07 D 321/06<br>C 07 D 319/08<br>C 07 D 317/34<br>C 07 D 317/72 |
| A | US-A-3 966 768 (PAWLOSKI)<br>* Spalten 1,2 *<br>----- | 1,7 | |
| | | | **RECHERCHIERTE SACHGEBIETE (Int. Cl.4)** |
| | | | C 07 D 319/00<br>C 07 D 317/00<br>C 07 D 321/00 |

Der vorliegende Recherchenbericht wurde für alle Patentansprüche erstellt

| Recherchenort | Abschlußdatum der Recherche | Prüfer |
|---|---|---|
| DEN HAAG | 10-05-1989 | FRANCOIS J.C.L. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)